# EUROPEAN PATENT APPLICATION

(11) **EP 3 971 258 A1**
(43) Date of publication of application: **23.03.2022**
(21) Application number: 21190852.0
(22) Date of filing: 26.05.2016
(51) Int. Cl.: C09K 5/06

(54) **PHASE CHANGE MATERIAL**

(30) Priority: 28.05.2015 GB 201509179
(62) Divisional of application: 16724917.6
(71) Applicant: DuPont Industrial Biosciences USA, LLC, Wilmington, Delaware 19805 (US)
(72) Inventor: NIELSEN, Bjarne, 8600 Silkeborg (DK); HENTZE, Hans-Peter, 8230 Abyhoj (DK)
(74) Representative: Mewburn Ellis LLP

(57) **Abstract**

This invention describes a phase change material being 1,3-propanediol ester where the 1,3-propanediol ester can be either a 1,3-propanediol monoester or a 1,3-propanediol diester. This invention further describes the use of 1,3-propanediol ester as a phase change material for releasing or absorbing latent heat during melting or crystallization. This invention also describes the use of the phase change material for use in non-food and food applications.

## Description

### FIELD OF THE INVENTION

The present invention relates to a phase change material being biobased and renewable and the use of the phase change material for non-food and food applications.

### BACKGROUND OF THE INVENTION

Phase Change Materials (PCM) are latent thermal storage materials that are capable of absorbing and releasing high amounts of latent heat during melting and crystallization, respectively. The thermal energy transfer occurs when a material is transformed from a solid to a liquid phase or from a liquid to a solid phase. During such phase changes, the temperature of the PCM material remains nearly constant as does the space surrounding the PCM material, the heat flowing through the PCM being "entrapped" within the PCM itself.

Phase change materials (PCMs) store and release significant amounts of thermal energy during melting and crystallization. They are used to control temperature in various applications like conditioning of buildings (e.g. Energain^{®} by DuPontTM), packaging, thermal protection of food, electronics, automotive applications, textiles and clothing, protection equipment, thermal energy storage or medical applications.

A wide range of inorganic and organic phase change materials are applied today. Inorganic phase change materials include salts (e.g. AlCl₃, LiNO₃, LiF), salt hydrates (e.g. KF·4H₂O, LiNO₃·3H₂O,MgCl₂·6H₂O) and various eutectic and non-eutectic mixtures (e.g. 66.6%urea+33.4%NH₄Br). Examples of organic phase change materials are paraffins (e.g. n-hexadecane, n-hexacozane, n-triacontane), fatty acids (e.g. caprylic acid, lauric acid, undecylenic acid), eutectic mixtures of fatty acids (*e.g.* 69%lauric acid/31%palmitic acid), monohydroxy alcohols (*e.g.* 1-tetradecanol), sugars (e.g. mannitol), ketones, ethers, amides, polymers and fatty acid esters (e.g. methyl palmitate, butyl stearate, ethylene glycol distearate).

This wide variety of phase change materials is used, as typically very specific properties of PCMs are required to enable a particular application. These properties include a specific melting temperature, a narrow melting temperature range and a high heat of fusion for high energy storage capacities. Depending on the particular application additional properties play an important role, too, such as hydrophilicity, hydrophobicity, flame-resistance, biodegradability or food-grade quality.

### OBJECT OF THE INVENTION

It is the object of the present invention to provide a new class of phase change materials which is bio-based, renewable and made from food-grade materials.

### DETAILED DESCRIPTION

This object is solved by a phase change material comprising 1,3-propanediol fatty acid esters. This object is further solved by using 1,3-propanediol fatty acid esters as phase change materials for releasing or absorbing latent heat during melting or crystallization.

This invention further describes a temperature regulating article comprising a phase change material, said phase change material being at least 1,3-propanediol fatty acid ester. Hereby, it is to be understood that the article may comprise other phase change materials than 1,3-propanediol fatty acid ester. In another embodiment, the article could also comprise different 1,3-propanediol fatty acid esters, optionally together with other phase change materials.

In a further embodiment the 1,3-propanediol fatty acid esters are 1,3-propanediol fatty acid monoesters. In a further use, the 1,3-propanediol fatty acid esters are 1,3-propanediol fatty acid monoesters.

In a still further embodiment, the 1,3-propanediol fatty acid esters are 1,3-propanediol fatty acid diesters. In a still further use, the 1,3-propanediol fatty acid esters are 1,3-propanediol fatty acid diesters.

The terms "heat of fusion" and "latent heat" are used interchangeably herein.

This invention describes for the first time a new class of phase change materials, which are 1,3-propanediol esters of fatty acids. 1,3-propanediol diesters and monoesters of fatty acids are fully bio-based, renewable and biodegradable. They are made from food-grade raw materials only and may therefore be used as food additives such as thermal protection of food ingredients.

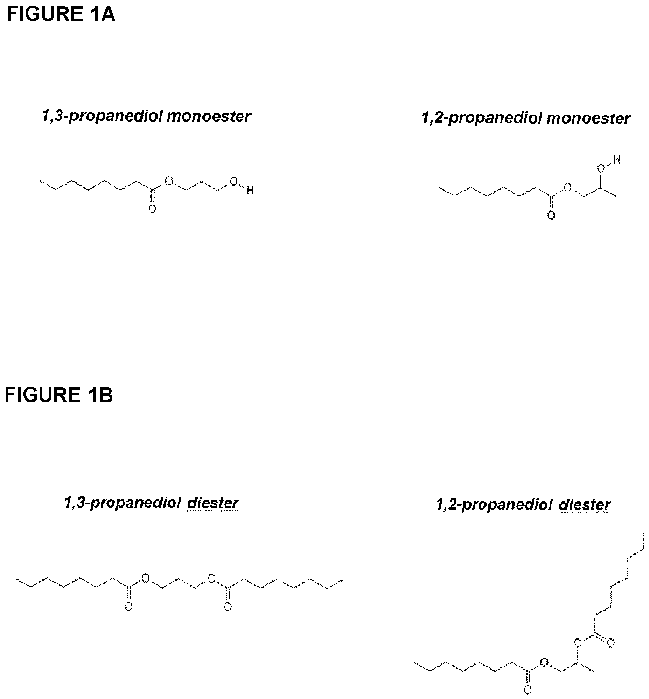

Phase change materials are known to release and absorb latent heat during melting or crystallization. 1,3-propanediol diesters and monoesters show characteristic PCM properties, such as high latent heat and low melting temperature ranges in contrast to 1,2-propanediol esters. This is due to the difference in the molecular structure of the fatty acid esters of 1,2-propanediol (propylene glycol) and 1,3-propanediol. The more linear structure of 1,3-propanediol monoesters (Figure 1A, left) allows a regular crystalline packing, which compared to the corresponding 1,2-propanediol monoesters (Figure 1A, right) enables more narrow melting ranges and high heat of fusion required for phase change materials. Similarly, the more linear structure of 1,3-propanediol diesters (Figure 1B, left) allows more regular crystalline packing compared to the corresponding 1,2-propanediol diesters (Figure 1B, right).

By temperature regulating article is to be understood any article which comprises a phase change material either as part of all the components of the article or as part of one or more components of the article.

A non-limiting list of articles is automotive articles such as engine cooling systems, construction materials such as wall panels, windows and floors, textiles such as jackets, shoes such as soles, protective equipment such as firefighter suits, articles for therapeutic hypothermia, electrical device, food packagings and different food formulations.

In one embodiment, the PCM comprises 1,3-propanediol diesters. In another embodiment, the PCM comprises 1,3-propanediol monoesters. In a further embodiment, the PCM comprises both 1,3-propanediol monoesters and 1,3-propanediol diesters.

The 1,3-propanediol esters are produced by direct esterification using an excess of fatty acids and 1,3-propanediol. After esterification residual free fatty acids and alcohol are removed by distillation. The remaining ester can be directly applied or further purified, e.g. by an additional distillation of the product. By way of example this could be performed as described in WO08/123845.

In a further embodiment, the phase change material is biobased. In a further use, the phase change material is biobased. Hereby, it is to be understood that the phase change material is produced from biological products in an environmentally friendly way. For instance, plant oil serves as the source of fatty acids and 1,3-propanediol is produced by fermentation of corn syrup (bioseparation of 1,3-propanediol). This process uses 40% less energy than the conventional 1,3-propanediol production and reduces greenhouse gas emissions by 20% (reference: http://brew.geo.uu.nl/BREWsymposiumWiesbaden11mei2005/WEBSITEBrewPrese ntations51105.PDF).

Bio-based and renewable 1,3-propanediol can be produced for example as described in WO1996/035796.

In a still further embodiment, the diester is a symmetric diester. In a further use, the diester is a symmetric diester. By symmetric diester is to be understood that the fatty acid diesters attached to the 1,3-diol are identical. Hereby, it is obtained that the molecule becomes symmetrical and this typically forms a more regular crystalline packing.

In a still further embodiment, the diester is a non-symmetric diester. In a further use, the diester is a non-symmetric diester. By a non-symmetric diester is to be understood that two different fatty acid esters are attached to one molecule of the 1,3-diol. In this way melting temperatures can be varied and finely adjusted.

In a still further embodiment, the 1,3-propanediol ester is a monoester. In this way the melting temperature of the PCM can be reduced compared to the corresponding 1,3-propanediol diesters.

In a further embodiment, said esters comprise fatty acids having a chain length of 2-24 carbon atoms. In a further use, said esters comprise fatty acids having a chain length of 2-24 carbon atoms. In a still further embodiment, said chain length is 8-22 carbon atoms. In a further use, said chain length is 8-22 carbon atoms.

The higher the melting temperature, the higher is the latent heat (see Table 1). Thus, by changing the chain length of the fatty acid esters attached to the 1,3-propanediol it is possible to change the characteristics of the phase change material. The longer the chain length of the fatty acid esters, the higher the melting temperature of the 1,3-propanediol ester will be and the more heat can be absorbed and released from the composition.

The fatty acids can be both saturated and unsaturated fatty acids such as but not limited to propionic acid, butyric acid, valeric acid, caproic acid, enathic acid, caprylic acid, pelargonic acid, capric acid, undecylic acid, luric acid, tridecylic acid myristic acid, pentadecylic acid, palmitic acid, margaric acid, staric acid, nonadecylic acid, arachidic acid, heneicosylic acid, behenic acid, tricosylic acid, lignoceric acid, α-linolenic acid, stearidonic acid, eicosapentaenoic acid, docosahexaenoic acid, linoleic acid, γ-linolenic acid, dihomo-γ-linolenic acid, arachidonic acid, docosatetraenoic acid, palmitoleic acid, vaccenic acid, paullinic acid, oleic acid, elaidic acid, gondoic acid, erucic acid, nervonic acid, mead acid.

In one embodiment the fatty acid esters attached to the 1,3-propanediol are either saturated or unsaturated fatty acids.

In a further embodiment, the fatty acid esters attached to the 1,3-propanediol are a saturated and an unsaturated fatty acid.

In a further embodiment, the phase change material comprises 1,3-propanediol fatty acid esters where some 1,3-propanediol fatty acid esters comprise saturated fatty acids and some comprise unsaturated fatty acids.

In a further embodiment, the phase change material comprises 1,3-propanediol fatty acid esters where some 1,3-propanediol fatty acid esters comprise saturated fatty acids and some comprise a mixture of saturated and unsaturated fatty acids.

In a further embodiment, the phase change material comprises 1,3-propanediol fatty acid esters where some 1,3-propanediol fatty acid esters comprise a mixture of unsaturated and saturated fatty acids and some comprise unsaturated fatty acids.

In a further embodiment, the phase change material comprises 1,3-propanediol fatty acid esters where some 1,3-propanediol fatty acid esters comprise saturated fatty acids, some comprise unsaturated fatty acids and some comprise a mixture of saturated and unsaturated fatty acids.

In a further embodiment, at least one of said fatty acids is saturated. In a further use, at least one of said fatty acids is saturated.

In one embodiment the fatty acids are saturated fatty acids such as but not limited to acetic acid, propionic acid, butyric acid, valeric acid, caproic acid, enathic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, including also functionalized fatty acids like 12-hydroxystearic acid.

The use of saturated fatty acids typically results in a higher crystallinity of the 1,3-propanediol esters compared to esters containing unsaturated fatty acids.

In a further embodiment, at least one of said fatty acids is unsaturated. In a further use, at least one of said fatty acids is unsaturated.

In one embodiment, the fatty acids are unsaturated fatty acids such as but not limited to α-linolenic acid, stearidonic acid, eicosapentaenoic acid, docosahexaenoic acid, linoleic acid, γ-linolenic acid, dihomo-γ-linolenic acid, arachidonic acid, docosatetraenoic acid, palmitoleic acid, vaccenic acid, paullinic acid, oleic acid, elaidic acid, gondoic acid, erucic acid, nervonic acid, mead acid.

In a still further embodiment, the fatty acids of said esters are linear. In a still further use, the fatty acids of said esters are linear. Linear fatty acids are more easily packed into a regular crystalline packaging. Hereby, the crystallinity is increased and more efficient PCM properties obtained.

In a still further embodiment, the fatty acid chains of said esters are linear and saturated. In a still further use, the fatty acid chains of said esters are linear and saturated.

In a further embodiment, said phase change material has a high heat of fusion of between 100-250 J/g when measured by DSC at a heating rate of 1°C/min. In a further use, said phase change material has a high heat of fusion of between 100-250 J/g when measured by DSC at a heating rate of 1°C/min.

In a still further embodiment, said heat of fusion is between 150-200 J/g when measured by DSC at a heating rate of 1°C/min. In a further use, said heat of fusion is between 150-200 J/g when measured by DSC at a heating rate of 1°C/min.

In a further embodiment, said phase change material has a high heat of fusion higher than 50 J/g when measured by DSC at a heating rate of 1°C/min. In a further use, said phase change material has a high heat of fusion higher than 50 J/g when measured by DSC at a heating rate of 1°C/min.

In a further embodiment, said phase change material has a high heat of fusion higher than 100 J/g when measured by DSC at a heating rate of 1°C/min. In a further use, said phase change material has a high heat of fusion higher than 100 J/g when measured by DSC at a heating rate of 1°C/min.

In a further embodiment, said phase change material has a high heat of fusion higher than 150 J/g when measured by DSC at a heating rate of 1°C/min. In a further use, said phase change material has a high heat of fusion higher than 150 J/g when measured by DSC at a heating rate of 1°C/min.

In a further embodiment, said phase change material has a high heat of fusion higher than 200 J/g when measured by DSC at a heating rate of 1°C/min. In a further use, said phase change material has a high heat of fusion higher than 200 J/g when measured by DSC at a heating rate of 1°C/min.

In a further embodiment, said phase change material has a phase transition temperature range of 1-20°C. In a further use, said phase change material has a phase transition temperature range of 1-20°C.

The phase transition temperature range is to be understood as the difference between left and right limit of the DSC curve preferably when measured at 1°C/min.

In a further embodiment, said phase change material has a phase transition temperature range of 1-15°C. In a further use, said phase change material has a phase transition temperature range of 1-15°C.

In a further embodiment, said phase change material has a phase transition temperature range of 1-10°C. In a further use, said phase change material has a phase transition temperature range of 1-10°C.

In a further embodiment, said phase transition temperature is 3-7°C. In a further use, said phase transition temperature is 3-7°C.

In a further embodiment, said phase change material further comprises additional thermal stabilizers. In a further use, said phase change material further comprises additional thermal stabilizers.

In one embodiment, the thermal stabilizers are antioxidants or blends of different antioxidants. Hereby thermal degradation, e.g. by free radicals or hydrolysis, is prevented or inhibited.

Examples of antioxidants for the thermal stabilization of 1,3-propanediol esters are tert-butylhydroquinone (TBHQ), pentaerythritol tetrakis(3-(3,5-di-tert-butyl-4-hydroxyphenyl)propionate), butylated hydroxytoluene (BHT), tris(2,4-ditert-butylphenyl)phosphite or propyl-3,4,5-trihydroxybenzoate.

In one embodiment, the thermal stabilizers are added in a concentration of 0.1 wt% to 3 wt%. In a further embodiment, the thermal stabilizers are added in a concentration of 0.2 wt% to 0.8 wt%. In a still further embodiment, the thermal stabilizers are added in a concentration of 0.4 wt% to 0.6 wt%.

In a further embodiment, said phase change material further comprises seed additives for reducing supercooling. In a further use, said phase change material further comprises seed additives for reducing supercooling.

In phase change materials supercooling should preferably be avoided since this lessens the effect of the phase change material. Supercooling is also known as undercooling where the temperature of a liquid or a gas is lowered below its freezing point without it becoming a solid.

Supercooling can be suppressed by addition of seed additives to the phase change material. 1,3-propanediol esters of short and medium chain length fatty acids show larger supercooling than 1,3-propanediol esters of long chain fatty acid esters. Addition of seed additives to the 1,3-propanediol esters of short and medium chain fatty acids effectively suppress supercooling hereof.

The seed additives can be particles or higher melting compounds. Seed particles are for instance polymer particles or silica particles. Higher melting compounds are waxes with higher melting temperatures, like paraffin waxes, ketones, ethers and esters. In a further embodiment, the higher melting compounds can be 1,3-propanediol esters with long chain fatty acids, e.g. with a fatty acid chain length of 18 carbon atoms or more.

This invention further describes use of a phase change material as described above in non-food applications such as automotive (e.g. engine cooling systems), construction materials (e.g. wall panels, windows, floors), textiles (e.g. jackets), shoes (e.g. soles), protective equipment (e.g. firefighter suits) and/or medical applications (e.g. therapeutic hypothermia).

The phase change material can further be used for applications such as thermal storage of solar energy, passive storage in bioclimatic building/architecture, cooling of engines, heating of water, maintenance of room temperature, thermal protection of electronic devices.

This invention further describes use of a phase change material as described above in food applications. In a further use, the phase change material is used for food packaging. In a further use, the phase change material is used for food formulations.

The use of a phase change material as described herein as a food additive is advantageous since the phase change material is made from food grade materials. It is commonly known that compounds often are transferred from the packaging itself to the food which is packaged. The use of a phase change material being a food grade material results in the packaging material being safer since the material may safely be ingested. Hereby, the food may also be thermally protected during transportation.

The food additive can also be added to the food component as such. Since the phase change material according to this invention is made from food grade materials, the phase change material may be ingested without any health risks. Hereby, the food grade PCM can be used in food formulation.

### BRIEF DESCRIPTION OF THE DRAWINGS

- Figure 1A: illustrates the molecular structure of the fatty acid monoesters 1,2-propanediol (propylene glycol) and 1,3-propanediol;
- Figure 1B: illustrates the molecular structure of the fatty acid diesters 1,2-propanediol (propylene glycol) and 1,3-propanediol;
- Figure 2: illustrates phase transition temperatures and heat temperatures and heat fusion of 1,3-propandiol fatty acid diesters;
- Figure 3A: illustrates a first measurement of melting temperature ranges and heat of fusion of 1,2-propandiol monolaurate;
- Figure 3B: illustrates a second measurement of melting temperature ranges and heat of fusion of 1,22-propandiol monolaurate;
- Figure 3C: illustrates a first measurement of melting temperature ranges and heat of fusion of 1,3-propanediol monolaurate;
- Figure 3D: illustrates a second measurement of melting temperature ranges and heat of fusion of 1,3-propanediol monolaurate;
- Figure 4A: illustrates a first measurement of melting temperature ranges and heat of fusion of 1,3-propandiol dibehanate;
- Figure 4B: illustrates a second measurement of melting temperature ranges and heat of fusion of 1,3-propandiol dibehanate;
- Figure 4C: illustrates a first measurement of melting temperature ranges and heat of fusion of 1,2-propanediol dibehenate;
- Figure 4D: illustrates a second measurement of melting temperature ranges and heat of fusion of 1,2-propanediol dibehenate;
- Figure 5A: illustrates DSC measurement (cooling curve) without seed addition (100 wt% 1,3-propandiol dicaprylate);
- Figure 5B: illustrates DSC measurement (cooling curve) with seed addition (97 wt% 1,3-propane diol dicaprylate + 3 wt% 1,3-propanediol dibehenate).

### EXAMPLES

### Material and methods

### Compounds

1,3-propanediol esters were produced by esterification of 1,3-propanediol with fatty acids similar to the synthesis of 1,3-propanediol dibehenate as described below.

2 kg of behenic acid, 186 g of 1,3-propanediol and 2.81 g of magnesium stearate were mixed by mechanical stirring at 180°C in a 5L 3-neck reaction flask equipped with a vigreux column, a condenser and a connection to a vacuum pump. At this temperature water started to form and condensed in the condenser. The reaction was performed in a nitrogen atmosphere. Heating was continued carefully until a reaction temperature between 200-210°C was reached. The reaction was kept at 205°C under nitrogen until an acid value of AV = 27 was reached. Subsequently, the reaction mixture was cooled to 175°C and neutralized with phosphoric acid. Stirring at this temperature was continued for 15 minutes, after which the reaction mixture was cooled to 100°C. Clarcell filter aid was added and the mixture was poured into a pre-heated Büchner funnel at 110°C to form a filter-cake. The remaining product was filtered at 100°C at a pressure of 500 mBar. Surplus of fatty acid and monoester were distilled off at 205°C at a pressure of 1×10⁻³ mBar. The resulting residue contained the 1,3-propanediol dibehenate product. The slightly yellowish product was further purified by a short path distillation under vacuum at 278°C (short path distillation unit KD-L5). The final product was applied as bulk material or as microbeads obtained by spray cooling.

### Differential Scanning Calorimetry (DSC) measurements

The characterization of the phase change materials has been performed using a dynamic scanning calorimeter (Metler Toledo DSC822). For each measurement aluminum standard pans with a volume of 40 microliter have been used to contain 1-3mg of the sample. The temperature profile was typically heating from 25°C to 80°C at 1°C/min (heat 1), C1: cooling from 80°C to 20°C at 1°C/min (cool 1), followed by subsequent heating from 20°C to 80°C at 1°C/min (heat 2). Depending on the type of PCM and the particular melting temperatures, alternatively higher or lower temperatures or heating and cooling rates have been applied (e.g. 10°C/min).

The melting temperatures and latent heat were determined after thermal equilibration from the 2^{nd} heat melting curves.

All measurements were performed in duplicates.

### II. Results

### Phase change material characteristics of 1,3 propanediol esters

Different 1,3-propanediol esters of saturated fatty acids with chain lengths of 8, 10, 12, 16, and 22 carbon atoms were obtained, i.e. 1,3-propanediol dibehenate, 1,3-propanediol dipalmitate, 1,3-propanediol dilaurate, 1,3-propanediol dicaprate, 1,3-propanediol dicaprylate, as well as 1,3-propanediol monolaurate.

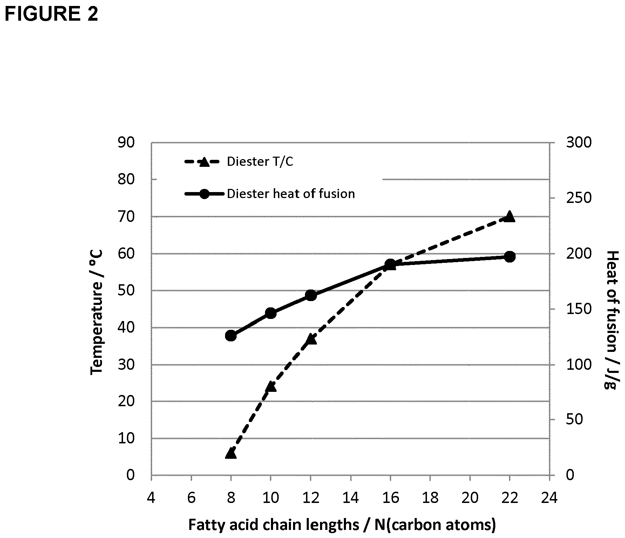

The products show typical PCM properties, such as high latent heat and narrow melting temperature ranges (Figure 2 and Table 1) as measured by DSC. This is most likely to be caused by a more regular packaging during crystallization owing to their more linear structure.

**Table 1: Phase transition temperatures and heat of fusion of 1,3-propandiol fatty acid esters**

| **1,3-propanediol ester** | **Fatty acid chain length** | **Melting temperature (°C)^{1,2}** | **Heat of fusion (J/g)^{1,3}** |
|---|---|---|---|
| 1,3-propanediol dicaprylate | 8 | 6 | 126 |
| 1,3-propanediol dicaprate | 10 | 24 | 146 |
| 1,3-propanediol dilaurate | 12 | 37 | 162 |
| 1,3-propanediol monolaurate | 12 | 24 | 155 |
| 1,3-propanediol dipalmitate | 16 | 57 | 190 |
| 1,3-propanediol dibehenate | 22 | 70.5 | 197 |

| | | | |
|---|---|---|---|
| ¹ Represent the average of measurements performed in duplicates. ² Melting temperature equals peak temperature of the DSC measurements. ³ Heat of fusion equals Normalized integral of the DSC measurements. | | | |

As an example Table 2 shows the values obtained in the separate two measurements of 1,3-propanediol dipalmitate by DSC.

**Table 2 shows duplicate measurements of 1,3-propanediol dipalmitate.**

| **Figure** | **Peak no.** | **Integral** | **Normalized integral** | **Onset** | **Peak** | **Left limit** | **Right limit** |
|---|---|---|---|---|---|---|---|
| 1,3-propanediol dipalmitate | 1^{st} run | -327.21 mJ | -189.14Jg^-1 | 56.49°C | 56.67°C | 45.48°C | 60.23°C |
| | 2^{nd} run | -355.42 mJ | -190.06Jg^-1 | 56.48°C | 56.69°C | 45.24°C | 60.20°C |

### Comparison between 1,3-propanediol monolaurate and 1,2-propanediol monolaurate

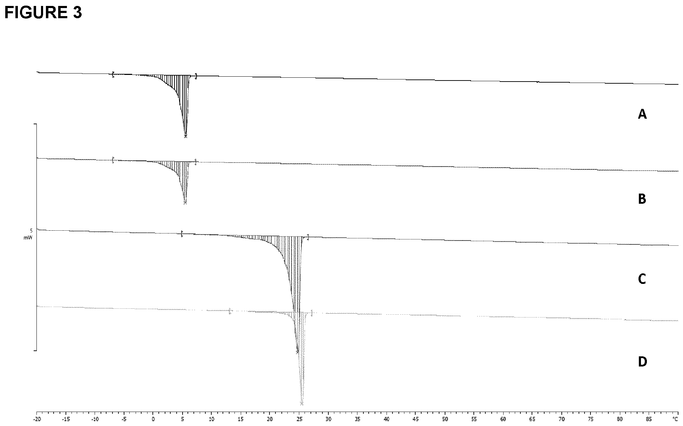

Comparative studies of 1,3-propanediol monolaurate and 1,2-propanediol monolaurate show that 1,2-propanediol monolaurate (Figure 3A+B and Table 3) exhibit a larger temperature melting range and a lower latent heat than 1,3-propanediol monolaurate (Figure 3C+D and Table 3). Thus, 1,2-propanediol monolaurate cannot be used as phase change material like 1,3-propanediol monolaurate.

**Table 3 shows the values of the integrals from Figure 3A-D.**

| **Figure** | **Peak no.** | **Integral** | **Normalized integral** | **Onset** | **Peak** | **Left limit** | **Right limit** |
|---|---|---|---|---|---|---|---|
| A* | 1 | -145.24 mJ | -110.03Jg^-1 | 3.82°C | 5.06°C | -7.06°C | 7.29°C |
| B* | 1 | -95.66 mJ | -112.54Jg^-1 | 3.86°C | 5.11°C | -7.06°C | 7.27°C |
| C** | 1 | -316.78 mJ | -156.05Jg^-1 | 22.54°C | 23.91°C | 4.77°C | 26.47°C |
| D** | 1 | -131.70 mJ | -154.94Jg^-1 | 24.22°C | 24.76°C | 12.96°C | 27.13°C |

| | | | | | | | |
|---|---|---|---|---|---|---|---|
| ^{*}1,2-propanediol monolaurate ^{**} 1,3-propanediol monolaurate | | | | | | | |

### Comparison between 1,3-propanediol dibehenate and 1,2-propanediol dibehenate

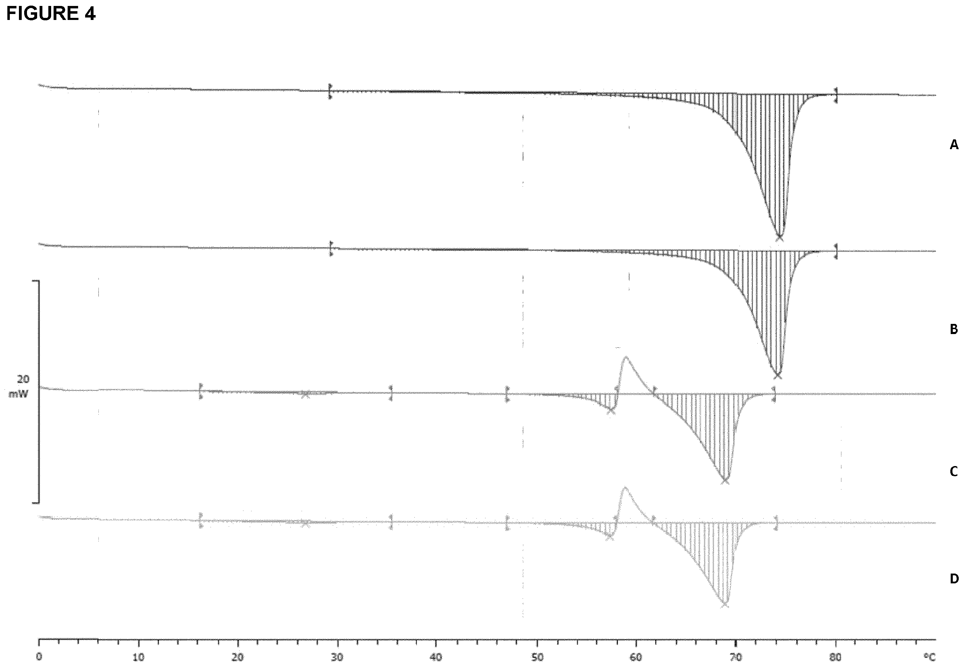

Comparative studies of 1,3-propanediol dibehanate and 1,2-propanediol dibehenate show that 1,2-propanediol dibehenate (Figures 4C+D and Table 4) exhibits a much larger temperature melting range and a lower latent heat than 1,3-propanediol dibehenate (Figures 4A+B and Table 4). Thus, 1,2-propanediol dibehenate cannot be used as phase change material like 1,3-propanediol dibehanate.

**Table 4 shows the values of the integrals from Figures 4A-D.**

| **Figure** | **Peak no.** | **Integral** | **Normalized integral** | **Onset** | **Peak** | **Left limit** | **Right limit** |
|---|---|---|---|---|---|---|---|
| A* | 1 | -751.48 mJ | -195.70Jg^-1 | 68.43°C | 70.77°C | 28.83°C | 79.78°C |
| B* | 1 | -631.92 mJ | -195.64Jg^-1 | 68.57°C | 71.06°C | 29.00°C | 79.85°C |
| C** | 1 | -14.92 mJ | -5.04Jg^-1 | 16.96°C | 26.51°C | 15.83°C | 35.11°C |
| | 2 | -44.49 mJ | -15.03Jg^-1 | 54.31°C | 56.76°C | 46.67°C | 57.75°C |
| | 3 | -388.13 mJ | -131.13Jg^-1 | 63.12°C | 66.66°C | 61.39°C | 73.60°C |
| D** | 1 | -14.53 mJ | -5.32Jg^-1 | 17.65°C | 26.53°C | 15.85°C | 35.13°C |
| | 2 | -39.25 mJ | -14.38Jg^-1 | 54.16°C | 56.67°C | 46.70°C | 57.68°C |
| | 3 | -363.01 mJ | -132.97Jg^-1 | 63.10°C | 66.72°C | 61.32°C | 73.77°C |

| | | | | | | | |
|---|---|---|---|---|---|---|---|
| ^{*}1,3-propanedioldibehenate ^{**} 1,2-propanediol dibehenate | | | | | | | |

### Addition of seed additives

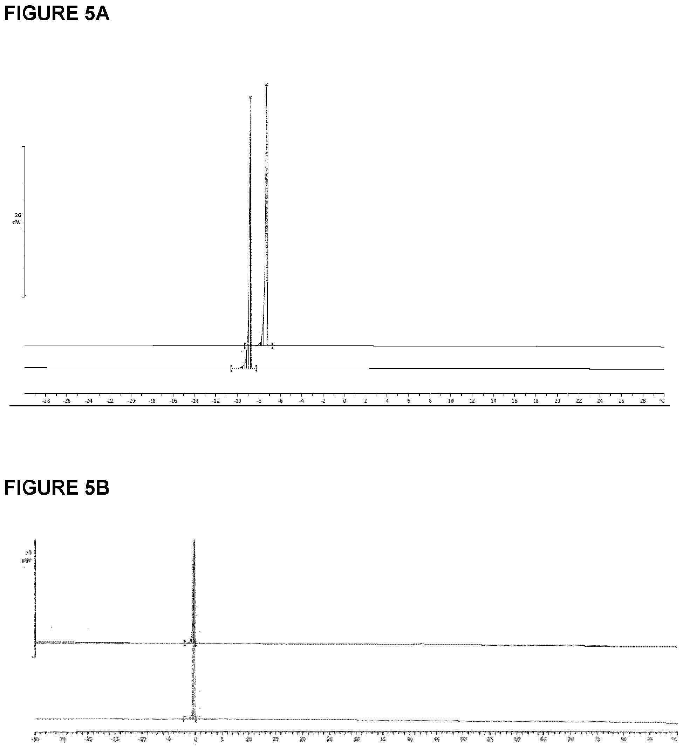

The higher the melting temperature, the higher is the heat of fusion. 1,3-propanediol diesters of short and medium chain length fatty acids show larger supercooling than long chain fatty acid esters (as shown in Figures 4 and 5). This is for instance the case when comparing the much larger supercooling of 1,3-propandiol dicaprylate (Figure 5) with the marginal supercooling of 1,3-propanediol dibehenate (Figure 4). Overall the samples show a solid-liquid transition over narrow temperature ranges and only little or no supercooling, which is in accordance with other fatty acid esters (see reference K. Pielichowska, Progress in Materials Science 65 (2014), page 79).

Supercooling of 1,3-propanediol dicaprylate was measured on pure caprylate (Figure 5A and Table 5) and on 1,3-propanediol dicaprylate comprising 3 wt% 1,3-propanediol dibehenate (Figure 5B and Table 5). The 1,3-propanediol dibehenate functions as a seed additive and effectively suppress supercooling of the short chain fatty ester diesters of 1,3-propanediol.

**Table 5 shows the values of the peaks from Figure 5A-B.**

| **Figure** | **Peak no.** | **Integral** | **Normalized integral** | **Onset** | **Peak** | **Left limit** | **Right limit** |
|---|---|---|---|---|---|---|---|
| A* | Left | 366.43 mJ | 122.14Jg^-1 | -8.63°C | 2.99°C | -10.72°C | -8.27°C |
| | Right | 369.67 mJ | 121.20Jg^-1 | -6.97°C | 3.98°C | -9.48°C | -6.78°C |
| B** | Top | 361.58 mJ | 120.13Jg^-1 | 0.17°C | 8.24°C | -2.23°C | -31.67e⁻⁰³°C |
| | Bottom | 374.84 mJ | 119.76Jg^-1 | 0.24°C | 8.71°C | -2.25°C | 3.40e⁻⁰³°C |

| | | | | | | | |
|---|---|---|---|---|---|---|---|
| ^{*}100 wt% 1,3-propanediol dicaprylate ^{**} 97 wt% 1,3-propanediol dicaprylate comprising 3 wt% 1,3-propanediol dibehenate | | | | | | | |

The following numbered paragraphs (paras.) contain further statements of various aspects of the present invention:
1. A phase change material comprising 1,3-propanediol fatty acid ester.
2. The phase change material according to clause 1, characterized in that the 1,3-propanediol fatty acid ester is a monoester.
3. The phase change material according to clause 1, characterized in that the 1,3-propanediol fatty acid ester is a diester.
4. The phase change material according to any of the preceding clauses, characterized in that said ester comprises fatty acids having a chain length of 2-24 carbon atoms.
5. The phase change material according to any of the preceding clauses, characterized in that at least one of said fatty acids is linear.
6. The phase change material according to clause 5, characterized in that at least one of said fatty acids is linear and saturated.
7. The phase change material according to any of the preceding clauses, characterized in that said phase change material has a high heat of fusion of between 100-250 J/g when measured by DSC at a heating rate of 1°C/min, such as between 150-200 J/g when measured by DSC at a heating rate of 1°C/min.
8. The phase change material according to any of the preceding clauses, characterized in that said phase change material has a high heat of fusion higher than 50 J/g when measured by DSC at a heating rate of 1°C/min, such as higher than 100 J/g when measured by DSC at a heating rate of 1°C/min, such as higher than 150 J/g when measured by DSC at a heating rate of 1°C/min, such as higher than 200 J/g when measured by DSC at a heating rate of 1°C/min.
9. The phase change material according to any of the preceding clauses, characterized in that said phase change material has a phase transition temperature range of between 1-20°C, such as a phase transition temperature range of between 1-15°C, such as a phase transition temperature range of between 1-10°C, such as a phase transition temperature of between 3-7°C.
10. The phase change material according to any of the preceding clauses, characterized in that said phase change material further comprises a thermal stabilizer.
11. The phase change material according to any of the preceding clauses, characterized in the said phase change material further comprises seed additives for reducing supercooling.
12. Use of 1,3-propanediol fatty acid ester as a phase change material as described in any of the clauses 1-11 for releasing or absorbing latent heat during crystallization or melting.
13. A use of a phase change material as described in any of the clauses 1-11 in non-food applications such as automotive, construction materials, textiles, shoes, protective equipment, medical applications, food applications such as food packaging and/or food formulations.
14. A temperature regulating article comprising a phase change material, said phase change material being as described in clauses 1-11.
15. A temperature regulating article according to clause 14, comprising a phase change material, said phase change material being described in clauses 1-11 having a composition that result in properties satisfying the need of the application wherein, said properties being the melting range and heat of fusion in J/g measured by DSC at a heating rate of 1°C/min.

## Claims

1. A phase change material comprising 1,3-propanediol fatty acid ester.

2. The phase change material according to claim 1, **characterized in that** the 1,3-propanediol fatty acid ester is a monoester.

3. The phase change material according to claim 1, **characterized in that** the 1,3-propanediol fatty acid ester is a diester.

4. The phase change material according to any of the preceding claims, **characterized in that** said ester comprises fatty acids having a chain length of 2-24 carbon atoms.

5. The phase change material according to any of the preceding claims, **characterized in that** at least one of said fatty acids is linear.

6. The phase change material according to claim 5, **characterized in that** at least one of said fatty acids is linear and saturated.

7. The phase change material according to any of the preceding claims, **characterized in that** said phase change material has a high heat of fusion of between 100-250 J/g when measured by DSC at a heating rate of 1°C/min, such as between 150-200 J/g when measured by DSC at a heating rate of 1°C/min.

8. The phase change material according to any of the preceding claims, **characterized in that** said phase change material has a high heat of fusion higher than 50 J/g when measured by DSC at a heating rate of 1°C/min, such as higher than 100 J/g when measured by DSC at a heating rate of 1°C/min, such as higher than 150 J/g when measured by DSC at a heating rate of 1°C/min, such as higher than 200 J/g when measured by DSC at a heating rate of 1°C/min.

9. The phase change material according to any of the preceding claims, **characterized in that** said phase change material has a phase transition temperature range of between 1-20°C, such as a phase transition temperature range of between 1-15°C, such as a phase transition temperature range of between 1-10°C, such as a phase transition temperature of between 3-7°C.

10. The phase change material according to any of the preceding claims, **characterized in that** said phase change material further comprises a thermal stabilizer.

11. The phase change material according to any of the preceding claims, **characterized in** the said phase change material further comprises seed additives for reducing supercooling.

12. Use of 1,3-propanediol fatty acid ester as a phase change material as described in any of the claims 1-11 for releasing or absorbing latent heat during crystallization or melting.

13. A use of a phase change material as described in any of the claims 1-11 in non-food applications such as automotive, construction materials, textiles, shoes, protective equipment, medical applications, food applications such as food packaging and/or food formulations.

14. A temperature regulating article comprising a phase change material, said phase change material being as described in claims 1-11.

15. A temperature regulating article according to claim 14, comprising a phase change material, said phase change material being described in claims 1-11 having a composition that result in properties satisfying the need of the application wherein, said properties being the melting range and heat of fusion in J/g measured by DSC at a heating rate of 1°C/min.
